(19) Europäisches Patentamt
European Patent Office
Office européen des brevets

(11) Veröffentlichungsnummer: **0 200 107 B1**

(12) **EUROPÄISCHE PATENTSCHRIFT**

(45) Veröffentlichungstag der Patentschrift: **16.10.91**

(51) Int. Cl.[5]: **C12N 11/00**

(21) Anmeldenummer: **86105330.4**

(22) Anmeldetag: **17.04.86**

# (54) Verfahren zur Immobilisierung gelöster Eiweissstoffe.

(30) Priorität: **27.04.85 DE 3515252**

(43) Veröffentlichungstag der Anmeldung:
**05.11.86 Patentblatt 86/45**

(45) Bekanntmachung des Hinweises auf die Patenterteilung:
**16.10.91 Patentblatt 91/42**

(84) Benannte Vertragsstaaten:
**AT CH DE FR GB IT LI NL SE**

(56) Entgegenhaltungen:
**DE-A- 2 527 884**
**DE-A- 2 905 671**
**GB-A- 2 082 188**
**GB-A- 2 129 809**

**Römpps Chemie Lexikon, Bd1, S. 78 (1979)**

(73) Patentinhaber: **Röhm GmbH**
**Kirschenallee Postfach 4242**
**W-6100 Darmstadt 1(DE)**

(72) Erfinder: **Krämer, Dieter, Dr.**
**An der Favorite 4**
**W-6500 Mainz(DE)**
Erfinder: **Plainer, Hermann, Dr.**
**Am Wembach 15**
**W-6107 Reinheim 1(DE)**
Erfinder: **Sprössler, Bruno, Dr.**
**Auf der Schmelz 93**
**W-6101 Rossdorf(DE)**
Erfinder: **Uhlig, Helmut, Dr.**
**Auf dem Wingert 4**
**W-6101 Rossdorf 1(DE)**
Erfinder: **Schnee, Reiner Dr.**
**Arheilger Woogstrasse 62**
**W-6100 Darmstadt(DE)**

**EP 0 200 107 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

### Technisches Gebiet

Die Erfindung betrifft ein Verfahren zur Immobilisierung von in Wasser gelösten Eiweißstoffen, insbesondere Enzymen, an festen Trägerkörpern in Gegenwart von Elektrolyten.

### Stand der Technik

Immobilisierte Enzyme sind schon durch die Vernetzung von gelösten Enzymen mit Glutardialdehyd erhalten worden. Dabei wurde beobachtet, daß steigende Ionenstärke die Vernetzung fördert, was mit der fällenden Wirkung von Elektrolyten erklärt wurde; vgl. K. Ogata et.al. Biochim. Biophys. Acta, Band 159 (1968), Seite 405 - 407.

Man hat auch Glutaraldehyd verwendet, um gelöste Enzyme an unlösliche Träger zu binden; vgl. A. Habeeb, Archives of Biochemistry and Biophysics, Band 119, (1967), Seite 264 - 268. Im Gegensatz zur trägerfreien Fällung und Vernetzung mit Glutardialdehyd wurde die Bindung an feste Träger stets bei niedrigen Elektrolytkonzentrationen durchgeführt.

Beim Verfahren gemäß DE-OS 33 36 257 werden dauerhaft immobilisierte Enzyme hergestellt, indem man einen porösen Träger, beispielsweise Diatomeenerde, mit einer Enzymlösung tränkt oder einen Träger mit einer Schicht des festen Enzyms überzieht und diese Zubereitungen aus einem Träger und einem gelösten oder festen Enzym in eine wäßrige Salzlösung einbringt und ein Vernetzungsmittel einwirken läßt. Die Salzlösung hat die Wirkung, daß von der Enzymzubereitung kein Enzym abgelöst wird und für die Immobilisierung verloren geht. Dieses Verfahren erfordert demnach grundsätzlich zwei getrennte Arbeitsschritte, nämlich die Herstellung einer Enzymzubereitung, die einen Träger und ein gelöstes oder lösliches Enzym enthält, und die Einbringung der Enzymzubereitung in ein Vernetzungsbad.

In EP 37667 ist die Bindung einer Pilzlactase an einen Trägerkörper aus makroporösen kationischen Ionenaustauscherharzen und in EP 26672 an Trägerkörper aus makroporösen amphoteren Ionenaustauscherharzen mittels Glutardialdehyd beschrieben. Bei beiden Verfahren werden Elektrolyte als Puffersubstanzen höchstens vor oder nach der Umsetzung mit Glutardialdehyd eingesetzt,während der Umsetzung selbst jedoch nur in geringen Konzentrationen, die 0,05 Mol/l in keinem Falle übersteigen, damit die ionische Adsorption nicht aufgehoben wird. Dagegen werden hohe Ionenkonzentrationen als Mittel erwähnt, um eventuell unvernetzt gebliebene Enzymmengen vom Träger abzulösen. Amphotere oder kationische Trägerharze können zu vielfältigen störenden Wechselwirkungen mit gelösten Bestandteilen der Substratflüssigkeiten führen und sind deshalb häufig nicht einsetzbar.

### Aufgabe und Lösung

Die vorliegende Erfindung löst die Aufgabe, die Immobilisierung von gelösten Eiweißstoffen an makroporösen Trägerkörpern mit hoher Aktivitätsausbeute auf einfache Weise in einem einstufigen Verfahren zu bewirken.

Es wurde gefunden, daß die Immobilisierung gelöster Eiweißstoffe an einem festen, makroporösen in Wasser nicht löslichen und höchstens schwach quellbaren Träger in einem einstufigen Verfahren gelingt, wenn man eine wäßrige Lösung des Eiweißstoffes, die eine Ionenstärke des Elektrolyten von mindestens 0,15 Mol/l aufweist, auf den von der wäßrigen Lösung umspülten Träger einwirken läßt, bis Adsorption des Eiweißstoffes an dem Träger erfolgt ist. Wenn die rein adsorptive Immobilisierung ausreichend ist, kann der beladene Träger einfach abgetrennt werden. In der Regel wird die Immobilisierung durch ein Vernetzungsmittel stabilisiert. Diese Vernetzung wird erfindungsgemäß unmittelbar in der elektrolythaltigen Lösung durchgeführt, ohne den eiweißbeladenen Träger vorher abzutrennen. Sowohl während der Adsorption als auch bei der meistens nachfolgenden Fixierung durch ein Vernetzungsmittel wird mit einem Überstand der elektrolythaltigen Lösung gearbeitet, so daß die suspendierten oder in einer Säulenschüttung angeordneten Trägerteilchen vollständig umspült sind.

### Vorteilhafte Wirkungen

Es ist überraschend, daß hohe Ionenstärken in dem hier beanspruchten Bereich die Immobilisierung des Eiweißstoffes auf dem makroporösen Trägerkörper fördern, obwohl hohe Ionenstärken sonst als ein Mittel gelten, um nicht kovalent verknüpfte Eiweißmoleküle von einem Träger abzulösen und in Gegenwart von Vernetzungsmitteln trägerfreie unlösliche Präzipitate zu bilden.

Die nachfolgende Tabelle zeigt die zunehmende Aktivitätsausbeute bei der Bindung von Penicillinamidase an Phenylsepharose mittels Glutardialdehyd bei steigender Ionenstärke. Das Bindungsverfahren entsprach der im Beispiel 5 angegebenen Arbeitsweise.

| Kaliumphosphatpuffer | | Aktivität des trägergeb. Enzyms | |
|---|---|---|---|
| Molarität (Mol/l) | Ionenstärke (Mol/l) | Int. Units pro Gramm Feuchtgewicht | Ausbeute in % immobil. Akt. |
| 0,01 | 0,03 | 64 | 36 |
| 0,05 | 0,15 | 103 | 51 |
| 0,10 | 0,30 | 128 | 72 |
| 0,50 | 1,50 | 170 | 92 |

Ohne Anwendung von Glutardialdehyd wird unter sonst gleichen Bindungsbedingungen überhaupt keine gebundene Aktivität festgestellt.

Die vorteilhafte Wirkung der erfindungsgemäß bevorzugten Arbeitsweise gegenüber der Immobilisierung an einem amphoteren Träger läßt sich am Beispiel der Immobilisierung von Hefelactase veranschaulichen. Nach dem Vergleichsversuch 1 der EP-A 26672 wird Hefelactase durch Glutardialdehyd bei pH 6,65 und 20 bis 22° C in einer Mengenausbeute von 64 % auf einem amphoteren Ionenaustauscher auf Basis eines Phenol-Formaldehyd-Harzes immobilisiert, jedoch ist die Aktivität so niedrig, daß praktisch gar keine Aktivitätsausbeute erreicht wurde.

Wird dagegen Hefelactase aus einer wäßrigen Lösung mit 111 g des Enzympräparats je Liter bei 23° C an ein perlförmiges, makroporöses, neutrales Trägerharz auf Basis von vernetztem Acrylamid mit Oxirangruppen als kupplungsaktiven Gruppen gebunden, so ergeben sich in Abhängigkeit von der angewandten Elektrolytkonzentration folgende Aktivitätsausbeuten:

| Elektrolyt-Konz. (Mol $K_2HPO_4$, $KH_2PO_4$/l) | Ionenstärke (Mol/l) | Aktivitätsausbeute in % der eingesetzten Aktivität |
|---|---|---|
| 0,5 | 1,21 | 3,5 |
| 1,0 | 2,42 | 19 |
| 1,25 | 3,02 | 51 |
| 1,5 | 3,33 | 55 |

Vor allem ist es überraschend, daß durch das erfindungsgemäße Verfahren auch an sehr unterschiedlichen, unspezifischen Trägerkörpern mit Leichtigkeit hohe absolute Aktivitäten und hervorragende Aktivitätsausbeuten erzielt werden, die sonst erst nach einer aufwendigen Aktivierung des Trägers erreichbar waren. Nach erfolgter Vernetzung bleibt der Eiweißstoff auch bei niedriger Ionenstärke an den Träger gebunden und kann - beispielsweise im Falle von Enzymen - ohne nennenswerte Aktivitätsverluste viele Male wiederverwendet werden.

## Gewerbliche Anwendung

Das Verfahren eignet sich zur Herstellung von festen makroporösen Körpern aller Art, die von Natur aus lösliche Eiweißstoffe in immobilisierter Form enthalten. Das wichtigste Anwendungsgebiet ist die Herstellung trägergebundener Enzyme. Daneben hat die Herstellung von Füllstoffen für die Affinitätschromatographie und die Herstellung von diagnostischen Testkörpern Bedeutung.

Die Eiweißstoffe,

die erfindungsgemäß gebunden werden können, sind solche, die bei einer Temperatur zwischen 0 und 60° C ausreichend wasserlöslich sind, um sie in gelöster Form mit dem Träger in Berührung bringen zu können. Die Erfindung ist nicht auf bestimmte Eiweißstoffe dieser Art beschränkt und umfaßt jedes biogene, wasserlösliche proteinhaltige Material. Obwohl Fälle des völligen Versagens des erfindungsgemäßen Verfahrens bisher nicht beobachtet wurden, kann es nicht ausgeschlossen werden, daß sich einzelne Eiweißstoffe nicht oder nur mit schlechterem Ergebnis als nach anderen Verfahren an Träger binden lassen. Es kann vorteilhaft sein, den Eiweißstoff durch Ionenzusätze oder, im Falle von Enzymen, durch Substratzusätze zu stabilisieren.

Bevorzugt sind Enzyme. Als Beispiel seien Amylasen, Proteasen, Amidasen, Pectinasen, Cellulasen, Hemi-cellulasen, Acylasen, Lactasen, Isomerasen, Invertasen und Oxidasen genannt. Beispiele für nicht enzymartige Eiweißstoffe sind Antikörper, Hormone mit Proteinstruktur und Enzyminhibitoren.

Der Träger

kann aus jedem in Wasser bzw. Elektrolytlösungen nicht löslichen Feststoff mit makroporöser Struktur bestehen. Geeignete Feststoffe mit makroporöser Struktur enthalten Poren von mindestens 10 nm Porendurchmesser und haben ein Porenvolumen über 0,1 $cm^3/g$, vorzugsweise über 0,5 $cm^3/g$. Bevorzugte Träger haben Porenweiten von 10 bis 100 nm, eine spezifische Oberfläche von 10 bis 500 $m^2/g$ und ein Porenvolumen von 1 bis 3 $cm^3/g$.

Die äußere Gestalt des Trägers ist für seine Wirkung nicht kritisch, wenn auch oberflächenreiche Systeme mit einer spezifischen Oberfläche von mindestens 1 $m^2/g$ vielfach bevorzugt sind, weil sie es gestatten, eine hohe Aktivitätsmenge des immobilisierten Eiweißstoffes auf kleinem Raum zu binden. Zu geeigneten Trägerkörpern gehören die inneren Oberflächen bzw. Beschichtungen von Gefäßen oder Rohren oder von festen Einbauten in Gefäßen oder Rohrleitungen, umspülbare oder durchströmbare Folien, Membranen, Papiere, Gewebe, Faservliese oder Faserpackungen oder andere Packungen oder Schichtungen von festen Körpern. Wenn zwischen diesen Körpern hinreichend große durchströmbare Räume liegen, können die Träger oder wenigstens die oberflächennahen Trägerschichten erheblich anquellen, ohne die Durchströmbarkeit zu beeinträchtigen. Bevorzugt werden kleinteilige Träger eingesetzt, worunter Trägerkörper mit einer Teilchengröße unter 10 mm, vorzugsweise im Bereich zwischen 0,1 und 5 mm verstanden werden. Besonders bevorzugt sind Perlen, d.h. kugelförmige Trägerkörper. Es ist vorteilhaft, wenn die kleinteiligen Träger in dem wäßrigen Medium, in dem sie angewendet werden, gar nicht oder nur wenig quellen, vorzugsweise auf nicht mehr als das doppelte Schüttvolumen der Trockensubstanz. Voraussetzung für die Eignung kleinteiliger Träger ist, daß sie sich in der elektrolythaltigen Lösung durch Rühren suspendieren oder in einer Säulenfüllung durchströmen lassen.

Bevorzugt sind Träger-Feststoffe, die nicht kationisch geladen sind. Darunter werden Feststoffe verstanden, die in ihrer Matrix oder wenigstens an ihrer zugänglichen Oberfläche keine oder jedenfalls nicht mehr als 0,1 m Äq/g kovalent gebundenen kationischen Gruppen oder basischen Gruppen, die durch Protonierung in kationische Gruppen übergehen können, also Ammonium- oder Aminogruppen, enthalten. Besonders bevorzugt sind ungeladene oder schwach anionisch geladene makroporöse Feststoffe. Als ungeladen werden Feststoffe angesehen, die keine oder nicht mehr als 0,1 Milli-äquivalent kovalent gebundene Carbonyl-, Carboxylat-, Sulfonat-, Amino- oder Ammoniumgruppen je Gramm trockenes Trägerharz enthalten. Schwach anionische Trägerharze können anionische Gruppen, wie kovalent gebundene Carboxyl- oder Carboxylatgruppen, in einer Konzentration bis zu 5m Äquiv. pro Gramm enthalten. Diese Werte gelten jedenfalls für die Harzsubstanz an der aktiven Oberfläche der Trägerteilchen.

Es hat sich aber herausgestellt, daß die oberflächliche Aussalzung des Proteins unter dem Einfluß der erhöhten Salzkonzentration auch an sehr hydrophilen Oberflächen, ja sogar an stark anionisch geladener Feststoffen stattfinden kann. Es sind daher auch solche Träger verwendbar.

Die Bindung der Eiweißstoffe wird durch hydrophobe Gruppen, z.B. gesättigte oder bevorzugt ungesättigte aliphatische Seitengruppen mit 2 oder mehr C-Atomen oder aromatische Reste, stark gefördert, wobei es weniger auf die Größe dieser Gruppen als auf ihre Flächendichte an der Oberfläche des Trägers ankommt. Bevorzugte Träger bestehen an der Oberfläche der Porenwandung oder durchgehend aus einem Polymermaterial mit mindestens 1 Gew.-% an Seitengruppen der genannten Art.

Grundsätzlich sind auch makroporöse anorganische Träger, wie Glasperlen oder Glasfasern (z.B. "Controlled Pore Glass"), Kieselsäure, Aluminiumoxid oder Aktivkohle geeignet. Organische Materialien, insbesondere Kunststoffe, haben den Vorteil, daß sich daraus Träger in definierter Form, z.B. Perlen, Fasern, Folien und Überzüge, leichter herstellen lassen. Der chemische Aufbau des organischen Trägerkörpers ist nicht kritisch, wenn auch im Einzelfall sorgfältig zu prüfen ist, mit welchem Träger sich das günstigste Ergebnis erreichen läßt.

Geeignete wasserunlösliche, makroporöse Kunstharze können z.B. von unpolarer Natur und unvernetzt oder vernetzt sein, wie z.B. Polymethylmethacrylat und andere Acrylesterpolymere, Polystyrol, Celluloseester, Phenol-Formaldehyd-Harze, Epoxidharze oder Polyolefine. Sie können auch polar und mehr oder weniger hydrophil, aber infolge von Vernetzung wasserunlöslich sein. Dazu gehören vernetzte Cellulose- und Stärkederivate, vernetztes Polyacrylamid oder Polymethacrylamid, vernetzte Polyhydroxyalkylester der Acryl- oder Methacrylsäure, vernetztes Polyvinylpyrrolidon oder nicht-basische Aminoplastharze.

Zur Verbesserung der Adsorptionseigenschaften ist es häufig von Vorteil, den Träger wenigstens oberflächlich etwas zu hydrophobieren. Man kann z.B. anorganische Träger, wie Siliciumdioxid, Bentonit oder poröse Gläser durch Umsetzung mit phenylfunktionellen Silanen oberflächlich mit Phenylgruppen ausrüsten. In entsprechender Weise können makroporöse organische Träger, wie Sepharose, mit Phenylglycidyläther phenyliert oder mit Alkylglycidyläther alkyliert werden. Außer Phenylresten haben im allgemeinen alle geradkettigen oder verzweigten Alkylreste mit 1 bis 18 C-Atomen eine adsorptionsfördernde Wirkung.

Eine besonders geeignete Gruppe von Trägerharzen in Form von Perlen oder Hohlperlen ist nach DE-B 22 37 316, 23 43 633 oder DE-A 27 22 751 zugänglich. Es handelt sich um vernetzte, wenig oder nicht quellfähige Polymerisate mit einer hydrophilen Matrix, die vorzugsweise zu einem wesentlichen Teil aus Acrylamid, Methacrylamid und vernetzend wirkendem Methylen-bis-acrylamid oder -methacrylamid aufgebaut sind. Sie enthalten gegenüber Eiweißstoffen bindungsaktive Gruppen, insbesondere Epoxidgruppen, weil diese durch Hydrolyse oder bei der Reaktion mit dem Eiweißstoff keine ionischen Gruppen bilden. Außerdem enthalten sie etwa 2 bis 5 Gew.-% freie Methacryloyl- bzw. Isopropanylgruppen, die von nur einseitig umgesetzten Vernetzungsmittelmolekülen herrühren. Bei Verwendung dieser Träger kann auf die Mitverwendung eines zusätzlichen Vernetzungsmittels verzichtet werden.

Der Elektrolyt

hat beim Verfahren der Erfindung vermutlich die Wirkung, daß er durch Strukturbildung von Wassermolekülen zu einer Art Übersättigung mit dem Eiweißstoff, jedenfalls zur Verminderung seiner Löslichkeit führt. Es ist häufig von Vorteil, den Elektrolyten erst nach der Auflösung des Eiweißstoffes zuzusetzen, da sich dieser sonst manchmal nur noch zögernd oder gar nicht auflöst.

Die Frage, welcher Elektrolyt in welcher Konzentration fällend wirkt, hängt von der Natur des Eiweißstoffes, seiner Konzentration und den Begleitstoffen ab und ist von Fall zu Fall auszuprobieren. Allgemein sind alle Elektrolyte von aussalzender Wirkung brauchbar, z.B. nach der Hofmeisterschen Reihe alle aussalzenden Anionen einschließlich Chlorid. Beispiele für die unterschiedliche Wirksamkeit von Elektrolyten gibt H.M. Rauen im "Biochemischen Taschenbuch", Teil 2, 2. Aufl., Seiten 56-57. Geeignet sind Elektrolyte mit einem positiven Wert von $K_s$ gemäß der Gleichung:

$$100 \frac{S}{S_o} = - K_s \cdot J$$

bzw. mit log $S_o = \beta$

$\log S = \beta - K_s \cdot J$

wobei S die Löslichkeit des Eiweißstoffes in Gegenwart und $S_o$ in Abwesenheit des Elektrolyten darstellt. Mehrwertige Metallkationen wirken häufig fällend oder inaktivierend, so daß einwertige Kationen, also vor allem Alkaliionen, vorzuziehen sind. Die Anionen haben einen deutlicheren Einfluß auf die Wirkung des Elektrolytzusatzes. Am besten sind Sulfate, Phosphate und Polyphosphate geeignet. Auch Carbonate, Chromate, Acetate, Citrate und Tartrate entfalten eine stark aussalzende Wirkung, sind aber bei empfindlichen Eiweißstoffen nicht immer anwendbar. Einwertige Anionen, wie Chloride oder Acetate, müssen in höherer Konzentration als mehrwertige Anionen eingesetzt werden, um die erforderliche Ionenstärke zu erreichen. Jene müssen, damit sie ihre aussalzende Wirkung entfalten können, in hoher Konzentration eingesetzt werden. Strukturbildende Neutralsalze, wie Ammonium-, Natrium- oder Kaliumsulfat, Ammonium-, Natrium- oder Kaliumhydrogenphosphat, sind wegen ihrer hohen Wirksamkeit, ihres geringen Preises und ihrer Unschädlichkeit für die meisten Eiweißstoffe am besten geeignet. Der pH-Wert der elektrolythaltigen

Eiweißlösung richtet sich nach der Empfindlichkeit des Eiweißstoffes und liegt vorzugsweise im Bereich von 4 bis 9.

Für die Wirksamkeit des Elektrolytzusatzes ist dessen Ionenstärke J maßgebend. Sie berechnet sich für eine wäßrige Lösung mit i Ionenarten nach der Formel

$$J = \frac{1}{2} \sum^{i} Z_i^{2} \; C_i$$

worin $C_i$ die Konzentration und $Z_i$ die Wertigkeit einer Ionenart darstellt. Beispielsweise ergibt sich für eine 1 m $K_2HPO_4$-Lösung aus

$$C_K = 2, \; Z_{K_{HPO4}} = 1, \; Z_{HPO_4} = 2$$

die Ionenstärke J = 3. In der Praxis lieger die Konzentrationen der bevorzugten Elektrolyte im Ionenstärken-Bereich zwischen 0,15 und 2 Mol/l. Vorzugsweise beträgt die Ionenstärke mindestens 0,3 Mol/l.

Die Immobilisierung

beginnt grundsätzlich mit einem Adsorptionsschritt. Auch wenn ein Vernetzungsmittel angewendet wird, geht der Vernetzungsreaktion der Adsorptionsschritt voraus. Man kann beide Schritte nacheinander in dieser Reihenfolge durchführen, d.h. zunächst den Eiweißstoff in Gegenwart des Elektrolyten an dem Träger adsorbieren und erst dann die vernetzende Kupplungskomponente hinzugeben. Diese Reihenfolge ist bevorzugt. Man kann aber auch beide Schritte verfahrensmäßig zusammenfassen und die Kupplungskomponente gleichzeitig mit dem Elektrolyten schon während der Adsorption einwirken lassen. Schließlich ist es auch in manchen Fällen möglich, zunächst den Träger mit der Kupplungskomponente allein umzusetzen und erst danach den Eiweißstoff und den Elektrolyten hinzuzufügen.

Die Adsorption des Eiweißstoffes findet in dem System aus einem Überstand der wäßrigen Lösung des Eiweißstoffes und aus dem Trägermaterial innerhalb von 0,1 bis 100Stunden, vorzugsweise 1 bis 10 Stunden, statt. Wesentlich ist, daß die Trägerteilchen in beiden Reaktionsphasen vollständig von der Elektrolytlösung umspült werden. Mit steigender Temperatur wird die Immobilisierung beschleunigt. Man arbeitet vorzugsweise bei der höchsten Temperatur, die der Eiweißstoff ohne Wirksamkeitsverlust verträgt. Obwohl man auch noch bei 0° C arbeiten kann, sind Temperaturen oberhalb 40° C, in manchen Fällen oberhalb 50° C besonders vorteilhaft.

Die zu immobilisierenden Eiweißstoffe können in einem breiten Konzentrationsbereich von beispielsweise 0,01 bis 30 %, bezogen auf das Gewicht der Lösung, und in einem breiten Bereich des Mengenverhältnisses zum Trägermaterial eingesetzt werden. Bei Enzymen werden hohe Bindungsausbeute und Produktaktivität angestrebt. Die Beladung liegt in der Regel über 20 mg Eiweißstoff je Gramm (trockenes) Trägermaterial, d.h. 2 Gew.-% oder mehr. Dagegen sind für immobilisierte Liganden zur Isolierung von Biomakromolekülen geringe Beladungsdichten vorteilhafter.

Die Immobilisierung kann in der Weise durchgeführt werden, daß der Träger in der Eiweißlösung aufgeschlämmt und nach Zusatz des Elektrolyten bis zum Abschluß des Verfahrens mäßig gerührt wird. Man kann auch die elektrolythaltige Eiweißlösung durch eine Schüttung des Trägers in einen Säulenreaktor strömen lassen und die Lösung längere Zeit umpumpen.

Die Immobilisierung besteht zunächst nur in der Adsorption des Eiweißstoffes am Träger. Bringt man den beladenen Träger anschließend in ein elektrolytärmeres Milieu, ist in vielen Fällen mit der Desorption des Eiweißstoffes zu rechnen. Das wird durch die nachfolgende kovalente Bindung der Eiweißmoleküle untereinander oder mit dem Träger unterbunden. Zu diesem Zweck wird in der zweiten Reaktionsphase eine Kupplungs- bzw. Vernetzungskomponente hinzugegeben, was verfahrensmäßig schon während der Adsorptionsstufe durchgeführt werden kann.

Die Kupplungskomponente muß wenigstens in einer zur Immobilisierung des Eiweißstoffes ausreichenden Menge in der wäßrigen Elektrolytlösung löslich sein. Wesentlich ist, daß diese Komponente in einem Überstand der Elektrolytlösung angewandt wird; d.h. die Lösung darf nicht von dem Trägermaterial

vollständig aufgesaugt werden. Je Milliliter feuchten Trägermaterials werden mindestens etwa 0,2 ml, vorzugsweise bis 10 ml Elektrolytlösung eingesetzt. Weiterhin muß die Kupplungskomponente in Gegenwart des Elektrolyten zur Einwirkung kommen, so daß dessen fällende Wirkung solange erhalten bleibt, bis der Eiweißstoff irreversibel immobilisiert ist.

Als Kupplungskomponente eignen sich wenigstens begrenzt wasserlösliche Verbindungen mit zwei oder mehr funktionellen Gruppen, mit denen sie mit korrespondierenden funktionellen Gruppen des Eiweißstoffes und gegebenenfalls auch des Trägers zu reagieren vermögen. Die irreversible Immobilisierung kann durch eine Vernetzung des Eiweißstoffes in sich selbst oder durch seine Vernetzung mit dem Träger zustandekommen. Geeignete, gegenüber Eiweißstoffen reaktive funktionelle Gruppen sind z.B. Aldehyd-, Epoxy-, Diazo-, Isocyanat- und Chlorameisenestergruppen. Weniger vorteilhaft sind in vielen Fällen Carbonsäureanhydrid- oder estergruppen, weil sie durch Bildung anionischer Carboxylatgruppen die elektrochemische Natur des Trägers stark verändern. Beispiele verwendbarer Kupplungskomponenten sind Diazobenzidin, Hexamethylendiisocyanat, Chlorameisensäureäthylester und als wichtigste Verbindung Glutardialdehyd. Man kann auch niedere wasserlösliche Polymere mit Molekulargewichten unter 100.000 einsetzen, wie Polyacrolein, Mischpolymere des Acryl- oder Methacrylamids mit Glycidylacrylat oder -methacrylat oder mit Acrolein oder N-Allylmethacrylamid.

Nicht unerwähnt sollen solche Kupplungskomponenten bleiben, die erst unter der Einwirkung von UV-Strahlung oder von Radikalbildnern (Redoxinitiatoren) kupplungsaktiv werden, z.B. Diallyläther oder Mischpolymerisate von Acryl- oder Methacrylamid mit p-Toluyl-hydroxyäthylmethacrylat.

Die zur irreversiblen Immobilisierung erforderliche Menge der Kupplungskomponente richtet sich nach ihrer Reaktionsfähigkeit gegenüber dem Eiweißstoff und gegebenenfalls dem Träger und kann beispielsweise im Bereich von 1 bis 100 Gew.-%, bezogen auf das Gewicht des Eiweißstoffes liegen. Die Reaktionsbedingungen der Kupplung unterscheiden sich im allgemeinen nicht von denen der Adsorption, so daß beide Vorgänge gleichzeitig unter den gleichen Bedingungen ablaufen können. Bei der Kupplung mit Glutardialdehyd sind in der Regel 0,1 bis 100 Std., vorzugsweise 2 Std. Reaktionsdauer bei Temperaturen zwischen 0 und 80 °C ausreichend.

Nach der Immobilisierungsreaktion wird in der Regel die Elektrolytlösung, die eventuell nicht gebundene Reste des Eiweißstoffes und der Kupplungskomponente enthält, von dem beladenen Träger abgetrennt. Dieser wird mit einer geeigneten Pufferlösung nachgewaschen und steht dann zur technischen Anwendung zur Verfügung.

Beispiel 1

Immobilisierte Glucose-Isomerase

Träer: Makroporöse, hochvernetzte Styrol/Divinylbenzol-Perlen mit einer inneren Oberfläche von ca. 200 $m^2/g$ und einem mittleren Porendurchmesser von 40 nm.

Enzym: Glucose-Isomerase, flüssiges Konzentrat aus einer Kultur von Streptomyces albus.

Aktivität: 1 g Enzym entickelt aus einer 0,1 molaren D-Glucose-Lösung bei pH = 7 und einer Temperatur von 70 °C in 60 Minuten 5 g D-Fructose.

Kupplung: Zuerst erfolgt die Adsorption des Enzyms an den makroporösen Träger. Dazu werden 10 g Träger mit 10 g Enzym in 50ml Salzlösung, enthaltend 12 % Natriumsulfat und 5 % Magnesiumsulfat, sowie 0,02 % Kobaltsulfat bei Raumtemperatur (23 °C) auf einer Rollbank gerollt. Die Ionenstärke der wäßrigen Lösung beträgt 4,18 Mol/l. Der pH-Wert wurde auf 7,0 eingestellt. Nach 20 Stunden werden 0,5 % Glutardialdehyd zugesetzt und weiter gerollt. Das adsorbierte Enzym wird dadurch "quervernetzt" und in den Poren festgehalten.

2 Std. später wird abgesaugt und gewaschen. Eine vergleichende Aktivitätsbestimmung ergibt eine Restaktivität im Filtrat von 45 %; im Träger werden 37 % wiedergefunden (= 37 % Aktivitätsausbeute ).

Anwendung: Die immobilisierte Glucose-Isomerase wird in einen Säulenreaktor gefüllt. Bei einer Temperatur von 60 °C wird bei einer Durchflußrate vom 7-fachen des Festbettvolumens pro Stunde eine 40 %ige Glucose-Lösung von pH = 7,5 zu 45 % zu Fructose isomerisiert.

Beispiel 2

Immobilisierte Aspergillus oryzae-Lactase

Träger: Vernetzter Polyacrylsäureester, mittlerer Porendurchmesser von 25 nm, innere Oberfläche 140$m^2/g$; Perlen von 0,3 - 1 mm Durchmesser.

Enzym: Aspergillus oryzae-Lactase, pulverförmiges Konzentrat. Aktivität = 30.000 U/g.

Kupplung: 10 g Träger werden mit 1 g Enzympräparat in 40 ml Salzlösung bei 35°C 8 Stunden geschüttelt. Die Salzlösung enthält 24 % Kalium-Chlorid und wurde auf pH = 5,0 eingestellt. Die wäßrige Lösung hat eine Ionenstärke von 3,21 Mol/l. Außerdem wurden zur Enzymstabilisierung 0,1 % Lactose zugesetzt. Anschließend wird abgekühlt und bei Raumtemperatur weitere 2 Stunden unter Zusatz von 0,5 % Glutardialdehyd geschüttelt. Dann wird abgesaugt und gewaschen. Die Lactase-Aktivitäten des Trägers und des Filtrats werden verglichen. Im Träger werden 34 % der eingesetzten Aktivität wiedergefunden, im Filtrat 11 %. Aktivitätsausbeute = 34%.

Anwendung: Die immobilisierte Aspergillus-Lactase wird in einen Säulenreaktor gefüllt. Bei einer Temperatur von 35°C wird eine 5 %ige Lactose-Lösung von pH = 4,5 bei einem Durchsatz von 40 Festbettvolumen pro Stunde zu über 90 % hydrolysiert. Nach 60 Tagen ist noch keine erkennbare Aktivitätsabnahme feststellbar.

Beispiel 3

Immobilisierte Hefelactase

Träger: Makroporöses, hochvernetztes Peripolymerisat auf Basis Methacrylamid/Methylen-bis-methacrylamid mit freien Epoxidgruppen (1,2 % Oxiran-Sauerstoff) und 2,2 % anhängenden Isopropenylgruppen, die auf die Poreninnenflächen konzentriert sind. Porenvolumen = 3,4 ml/g, mittlerer Porendurchmesser = 20 nm. Die Herstellung dieses Trägers ist im Beispiel 2 der DE-A 27 22 751 beschrieben.

Die Kupplung des adsorbierten Enzyms erfolgt hier kovalent durch die Epoxidgruppen, zugleich mit seiner Adsorption unter dem Einfluß der hohen Salzkonzentration.

Enzym: Hefelactase von Saccharomyces (Kluyveromyces) lactis, Flüssigpräparat, 5000 neutrale Lactase-Einheiten (NLU).

Kupplung: 10 g Träger wird mit 10 g Enzym bei Raumtemperatur (23°C) in 80 g Salzlösung geschüttelt. Die Salzlösung enthält 16 % Dikaliumhydrogenphosphat, 7,9 % Kaliumdihydrogenphosphat und zur Stabilisierung des Enzyms 20 ppm Mn(II)-Chlorid 4 $H_2O$. Sie hat eine Ionenstärke von 3,34 Mol/l. Nach 72 Std. wird abgesaugt, gewaschen und die Lactase-Aktivität des Filtrats mit der des Trägers verglichen. 55 % der eingesetzten Aktivität werden am Träger wiedergefunden, 14 % im Filtrat.

Die Aktivitätsausbeute ist also mit 55 % bei diesem empfindlichen Enzym sehr hoch.

Anwendung: Die gekuppelte Hefelactase wird in einen Säulenreaktor gefüllt. Bei einer Temperatur von 7°C wird 20 Tage lang Magermilch (0,3 % Fett) bei einer Durchflußrate von 55 Festbettvolumen pro Stunde durchgeleitet. Die in der Milch enthaltende Lactose wird anfänglich zu 65 % zu Glucose und Galaktose hydrolysiert, nach 20 Tagen noch zu 50 %.

Beispiel 4

Immobilisierte Aminosäure-Acrylase

Träper: Makroporöses, hochvernetztes Perlpolymerisat auf Basis Methacrylamid/Methylen-bis-methacrylamid mit freien Epoxidgruppen (1,2 % Oxiran-Sauerstoff) und 2,2 % anhängenden Isoprepenylgruppen, die auf die Poreninnenflächen konzentriert sind. Porenvolumen = 3,4 ml/g, mittlerer Porendurchmesser = 20 nm. Die Herstellung dieses Träger ist im Beispiel 2 der DE-A 27 22 751 beschrieben.

Enzym: Pulverförmiges Aminosäure-Acylase-Konzentrat aus einem Aspergillus-Stamm.
Aktivität: 23.000 u/g, Substrat = Acetyl-D,L-Methionin.

Kupplung: 10 g Träger werden mit 20 g Enzympräparat bei 35°C in 80 ml Salzlösung geschüttelt. Die Salzlösung enthält 14,2 % Natriumsulfat sowie 24 ppm Kobalt(II)-chlorid • 6 $H_2O$ und ist auf pH = 7,0 eingestellt. Ihre Ionenstärke beträgt 3 Mol/l ohne Berücksichtigung des Salzgehalts des Enzympräparats.

Nach 8 Stunden wird der Träger abgesaugt und gewaschen. 61 % der eingesetzten Aktivität werden im Träger wiedergefunden, im Filtrat 1 %. Es wurde somit eine Aktivitätsausbeute von 61 % erzielt.

Beispiel 5

Bindung von Penicillinamidase an verschiedene Träger

Jeweils 3,5 g feuchtes Trägermaterial gemäß Tabelle I werden 5 mal mit dem jeweils fünffachen Volumen entsalzten Wassers gewaschen und auf einer Glasfritte abgesaugt. Dann wird das Trägermaterial

mit 6,8 ml einer Enzymlösung, enthaltend 676 Internat. Units Penicillin-Amidase aus E.coli in 0,5 M K-Phosphatpuffer (pH 7,5, mit 0,1 % $NaN_3$) zwei Stunden bei etwa 21 °C geschüttelt. Die Ionenstärke der Pufferlösung beträgt 1,5 Mol/l. Nach Zugabe von 0,136 ml einer 25 %igen wäßrigen Glutardialdehyd-Lösung, die über Ionenaustauscherharz (Amberlite A 21) stabilisiert war, wird zwei Stunden weiter geschüttelt. Dann wird das beladene Trägermaterial mit Wasser auf eine Glasfritte gebracht und dreimal mit 1 M NaOH und zweimal mit 0,05 M Na-Phosphatpuffer (pH 7,5, mit 0,1 % $NaN_3$) nachgewaschen.

Die enzymatische Aktivität wurde gegen Penicillin-G K (crude) als Substrat durch alkalimetrische Titration bei pH 7,5 bestimmt. Dazu wurden jeweils 20 ml der 2 %igen Substratlösung in 0,05 M Na-Phosphatlösung pH 7,5 eingesetzt und bei 37 °C mit 0,5 M Natronlauge automatisch titriert. Die Ergebnisse sind in Tabelle I angegeben.

Tabelle I

| Trägermaterial: Chem. Zusammensetzung | (Handelsbez.) | Aktivität der immobilisierten PC-Amidase: U/g Feuchtgewicht | Aktivitätsausbeute |
|---|---|---|---|
| a) vernetzte Agarose | (Sepharose-CL-4B*) | 96 | 58 |
| b) octylierte, vernetzte Agarose | (Octyl-Sepharose-CL-4B*) | 98 | 53 |
| c) phenylierte vernetzte Agarose | (Phenyl-Sepharose-CL-4B*) | 168 | 91 |
| d) vernetzte Agarose, mit Diäthylaminoäthylgruppen subst. (kationisch) | (DEAE-Sepharose-CL-6B*) | 84 | 61 |
| e) carboxymethylierte vern. Agarose (anionisch) | (CM-Sepharose CL-6B*) | 94 | 48 |
| f) Phenoxyacelylcellulose | | 77 | 59 |
| g) Oxiran-polyacrylamidharz (nach DE 27 22 751, Beispiel 1), umgesetzt mit Benzylmercaptan | | 172 | 85 |
| h) Polymethacrylimid-Schaumstoff, gemahlen | (Rohacell WF, Röhm GmbH) | 70 | 45 |
| i) vernetztes Polymethylmethacrylat-Glykoldimethacrylat-Mischpolymerisat**) Gewichtsverhältnis 90 : 10 | | 35 | 25 |

| Trägermaterial Chem. Zusammensetzung | (Handelsbez.) | Aktivität der immobilisierten PC-Amidase U/g Feuchtgewicht | Aktivitätsausbeute |
|---|---|---|---|
| j) vernetztes Polymethylmethacrylat-Glykoldimethacrylat-Mischpolymerisat**) Gewichtsverhältnis 80 : 20 | | 55 | 37 |
| k) vernetztes Polymethylmethacrylat-Glykoldimethacrylat-Mischpolymerisat**) Gewichtsverhältnis 60 : 40 | | 43 | 33 |
| l) vernetztes Polystyrol mit 10 % Divinylbenzol**) | | 57 | 42 |
| m) Poröses Glas Porenvolumen 0,75 $cm^3/g$ mittl. Porendurchm. 17 nm innere Oberfläche 107 $m^2/g$ | (Controlled Pore Glass-10) | 105 | 60 |

*) eingetr. Warenzeichen der Pharmacia AB, Uppsala, Schweden.

**) Herstellungsverfahren: In einem Rührkessel wird eine Lösung von 1 Gew.-Teil (T) Polyvinylalkohol in 320 T Wasser auf 50°C erwärmt und darin unter Rühren eine Mischung von 100 T Monomeren (Methylmethacrylat + Glykoldimethacrylat bzw. Styrol + Divinylbenzol), 60 T n-Heptan, 1,4 T Dibenzoylperoxid tröpfchenförmig verteilt. Während der 4-stündigen Polymerisationszeit wird die Temperatur durch Kühlung auf max. 75°C gehalten. Danach wird in 1 Std. bei 36°C das Lösungsmittel abdestilliert. Die entstandenen Polymerisatperlen werden nach dem Erkalten abfiltriert.

Beispiel 6

Bindung von Trypsin an Phenyl-Sepharose

3,5 g Phenyl-Sepharose (Feuchtgewicht) werden wie im Beispiel 1 vorbehandelt. Dann werden 150 mg Trypsin gelöst in 6,8 ml 0,5 M Kaliumphosphatpufferlösung (pH 7,5, mit 0,1 % $NaN_3$) zugegeben und 2 Stunden lang bei 23 °C geschüttelt. Die Ionenstärke beträgt 1,5 Mol/l. Die Fixierung des adsorbierten Enzyms und die Nachbehandlung werden wie im Beispiel 1 durchgeführt.

Die enzymatische Aktivität wurde gegen Kasein und gegen N-Benzoyl-l-argininäthylester- hydrochlorid (BAEE) als Substrate bestimmt.

Ergebnisse: gegen Kasein 8,3 U/g Feuchtgewicht gegen BAEE 311 U/g Feuchtgewicht.

Beispiel 7

Herstellung von trägergebundenen Lactase-Präparaten

Jeweils 1 g einer Aspergillus oryzae-Lactase (Handelsbezeichnung "Lactase-Präparat 2214 C Konz.", Röhm GmbH) mit 30.000 U/g wird in 40 ml einer 0,7 M $Na_2SO_4$-Lösung der Ionenstärke 2,1 Mol/l, pH 5,5, gelöst und die Lösung mit jeweils 10g der in der Tabelle angegebenen Trägerharze versetzt und 20 Std.1 bei Raumtemperatur geschüttelt. Dann werden 0,8 ml 25 %ige wäßrige Glutardialdehydlösung zugegeben und 2 Std. bei Raumtemperatur geschüttelt. Die Präparate werden abfiltriert, gewaschen und die Aktivität des immobilisierten und des im Filtrat zurückgebliebenen Enzyms bestimmt und in Prozent auf die eingesetzte Aktivität ausgedrückt. Ergebnisse in Tabelle II:

Tabelle II

| Trägermaterial Chem. Zusammensetzung | (Handelsbez.) | Aktivität immobil. Lactase Aktiv-Ausbeute % | Restaktivität im Filtrat % |
|---|---|---|---|
| a) schwach basischer Ionenaustauscher Basis Styrol/Divinylbenzol | (Amberlite IRA 93[1]) | 17,5 | 0 |
| b) makroporöses Adsorberharz Basis Polyacrylsäureester | (Amberlite XAD 7[1]) | 29,5 | 18 |
| c) makroporöses Phenol-Formaldehyd-Adsorberharz schwach basisch | (Duolite S 561[2]) | 24,0 | 5 |
| d) makroporöses Phenol-Formaldehyd-Adsorberharz schwach basisch | (Duolite S 587[2]) | 26,0 | 4 |
| e) makroproöses Phenol-Formaldehyd-Adsorberharz nicht-ionisch | (Duolite S 761[2]) | 22,0 | 6 |
| f) makroporöses Glas Porenvolumen 0,75 $cm^3/g$ mittl. Porendurchm. 17 nm innere Oberfläche 107 $m^2/g$ | (Controlled Pore Glass CPG 10) | 42,4 | 8 |
| g) Polymethacrylimid-Schaumstoff, gemahlen | (Rohacell[3]) | 31,C | 0 |
| h) Hydroxylapatit (bas. Calciumphosphat) gemahlen | - | 16,5 | 4 |

1) eingetr. Warenzeichen der Röhm & Haas Co., Philadelphia, USA

2) eingetr. Warenzeichen der Duolite International Co.

3) eingetr. Warenzeichne der Röhm GmbH, Darmstadt

## Patentansprüche

1. Verfahren zur Immobilisierung eines gelösten Eiweißstoffes an einen festen, makroporösen, in Wasser nicht löslichen Träger in Gegenwart eines Elektrolyten,

dadurch gekennzeichnet,

daß man mindestens 0,2 ml einer wäßrigen Lösung des Eiweißstoffes, die eine Ionenstärke des Elektrolyten von mindestens 0,3 Mol/l aufweist, auf einen Milliliter des von einem Überstand der wäßrigen Lösung umspülten feuchten Trägers einwirken läßt, bis Adsorption des Eiweißstoffes an den Träger erfolgt ist, und daß man während oder nach der Adsorption des Eiweißstoffes an den Träger eine wasserlösliche, die Vernetzung des an den Träger adsorbierten Eiweißstoffes bewirkende Kupplungskomponente zu der elektrolythaltigen Lösung zusetzt.

2. Verfahren nach Anspruch 1, dadurch gekennzeichnet, daß ein Träger ohne oder mit nicht mehr als 0,1 mÄq/g (mMol/g) kovalent gebundenen kationischen Gruppen eingesetzt wird.

3. Verfahren nach Anspruch 2, dadurch gekennzeichnet, daß ein Träger mit höchstens 5 mÄquiv. (mMol) kovalent gebundenen anionischen Gruppen je Gramm Trockensubstanz eingesetzt wird.

4. Verfahren nach Anspruch 3, dadurch gekennzeichnet, daß ein Träger mit höchstens 0,1 mÄquiv. (mMol) kovalent gebundenen ionischen Gruppe je Gramm Trockensubstanz eingesetzt wird.

5. Verfahren nach den Ansprüchen 1 bis 4, dadurch gekennzeichnet, daß ein Träger verwendet wird, der in wäßrigen Medien höchstens auf das doppelte Volumen quillt.

6. Verfahren nach Anspruch 5, dadurch gekennzeichnet, daß ein Träger in Form kleiner Teilchen, insbesondere in Form von Perlen verwendet wird.

## Claims

1. Process for immobilising a dissolved protein on a solid, macroporous, water-insoluble carrier in the presence of an electrolyte, characterised in that at least 0.2 ml of an aqueous solution of the protein substance, which has an ionic strength of the electrolyte of at least 0.3 mol/l, is allowed to act on one millilitre of the moist carrier, which has been rinsed with the supernatant of the aqueous solution, until the protein substance has been adsorbed into the carrier, and during or after the adsorption of the protein substance onto the carrier a water-soluble coupling component which brings about the crosslinking of the protein substance adsorbed onto the carrier is added to the electrolyte-containing solution.

2. Process according to claim 1, characterised in that a carrier is used having no or not more than 0.1 mEq/g (mMol/g) of covalently bound cationic groups.

3. Process according to claim 2, characterised in that a carrier is used having at most 5 mequiv. (mMol) covalently bound anionic groups per gram of dry matter.

4. Process according to claim 3, characterised in that a carrier is used having at most 0.1 mequiv. (mMol) covalently bound ionic groups per gram of dry matter.

5. Process according to claims 1 to 4, characterised in that a carrier is used which swells to at most twice its volume in aqueous media.

6. Process according to claim 5, characterised in that a carrier is used in the form of small particles, more particularly beads.

## Revendications

1. Procédé pour l'immobilisation d'une matière albuminoïde en solution sur un support solide, macroporeux, non soluble dans l'eau, en présence d'un électrolyte, caractérisé en ce qu'on fait agir au moins 0,2 ml d'une solution aqueuse de la matière albuminoïde, qui présente une force ionique de l'électrolyte d'au moins 0,3 mole/l, sur 1 ml du support humide baigné d'une couche couvrante de la solution aqueuse, jusqu'à ce que l'adsorption de la matière albuminoïde sur le support ait été effectuée, et en ce qu'on ajoute à la solution contenant l'électrolyte, pendant ou après l'adsorption de la matière

albuminoïde sur le support, un composant de copulation hydrosoluble qui provoque la réticulation de la matière albuminoïde adsorbée sur le support.

2. Procédé selon la revendication 1, caractérisé en ce qu'on utilise un support dépourvu de groupements cationiques fixés par une liaison covalente ou n'en renfermant pas plus de 0,1 meq/g (mmol/g).

3. Procédé selon la revendication 2, caractérisé en ce qu'on utilise un support renfermant au maximum 5 meq (mmol) de groupements anioniques fixés par une liaison covalente par gramme de substance sèche.

4. Procédé selon la revendication 3, caractérisé en ce qu'on utilise un support renfermant au maximum 0,1 meq (mmol) de groupements ioniques fixés par une liaison covalente par gramme de substance sèche.

5. Procédé selon l'une quelconque des revendications 1 à 4, caractérise en ce qu'on utilise un support qui gonfle au maximum au double de son volume dans des milieux aqueux.

6. Procédé selon la revendication 5, caractérisé en ce qu'on utilise un support sous forme de petites particules, en particulier sous forme de perles.